# NEW EUROPEAN PATENT SPECIFICATION

(11) **EP 0 998 291 B2**
(45) Date of publication and mention of the opposition decision: **06.07.2011**
(45) Mention of the grant of the patent: 11.07.2007
(21) Application number: 98935126.7
(22) Date of filing: 16.07.1998
(51) Int. Cl.: A61K 31/64, A61K 31/44, A61P 3/10

(54) **TREATMENT OF DIABETES WITH THIAZOLIDINEDIONE AND SULPHONYLUREA**
BEHANDLUNG DER DIABETES MIT THIAZOLIDINDIONE UND SULPHONYLUREA
TRAITEMENT DU DIABETE AVEC DU THIAZOLIDINEDIONE ET DE LA SULFONYLUREE

(30) Priority: 18.07.1997 GB 9715306
(43) Date of publication of application: 10.05.2000
(73) Proprietor: SMITHKLINE BEECHAM PLC, Brentford, Middlesex TW8 9GS (GB)
(72) Inventor: BUCKINGHAM, Robin, Edwin SmithKline Beecham Pharma, Harlow Essex CM19 5AW (GB); SMITH, Stephen, Alistair SmithKline Beecham Pharma, Harlow, Essex CM19 5AW (GB)
(74) Representative: West, Vivien
(86) International application number: PCT/GB1998/002109
(87) International publication number: WO 1999/003476

(56) References cited:
- EP-A- 0 749 751
- EP-A- 0 861 666
- WO-A-93/03724
- WO-A-98/36755
- KELLEY D.E. ET AL: "Troglitazone" CURRENT OPINION IN ENDOCRINOLOGY AND DIABETES, 1998, 5/2 (90-96), XP002080820 United States
- SCHEEN AJ ET AL: "Oral antidiabetic agents. A guide to selection." DRUGS, FEB 1998, 55 (2) P225-36, XP002080821 NEW ZEALAND
- IWAMOTO Y ET AL: "EFFECT OF COMBINATION THERAPY OF TROGLITAZONE AND SULPHONYLUREAS INPATIENTS WITH TYPE 2 DIABETES WHO WERE POORLY CONTROLLED BY SULPHONYLUREA TERAPY ALONE" DIABETIC MEDICINE, vol. 13, no. 4, April 1996, pages 365-370, XP002064289
- SCHEEN AJ: "Drug treatment of non-insulin-dependent diabetes mellitus in the 1990s. Achievements and future developments." DRUGS, SEP 1997, 54 (3) P355-68, XP002080822 NEW ZEALAND
- AMERICAN DIABETES ASSOCIATION MEETING LOUISIANA 2003, Abstract No. 1946
- PATEL ET AL.: 'BRL 49653 (a thiazolidinedione) Improves Glycemic Control in NIDDM Patients' DIABETES vol. 46, no. 1, May 1997, page 150A
- MILLER ET AL.: 'BRL 49653 (a thiazolidinedione) is well tolerated and has no' DIABETES vol. 46, no. 1, May 1997, page 96A
- WILLSON ET AL.: 'The structure-activity relationship between peroxisome proliferator-activated receptor y agonism and the antihyperglycemic activity of thiazolidinediones' JOURNAL OF MEDICINAL CHEMISTRY vol. 39, no. 3, 1996, pages 665 - 668
- CANTELLO ET AL.: '¬¬-(heterocyclylamino)alkoxy|benzyl|-2,4-t hiazolidinediones as potent antihyperglycemic agents' JOURNAL OF MEDICINAL CHEMISTRY vol. 37, 1994, pages 3977 - 3985
- BRESSLER ET AL.: 'Pharmacological regulation of blood glucose levels in non-insulin-dependent diabetes mellitus' ARCH. INTERN. MED. vol. 157, April 1997, pages 836 - 848
- LEITER: 'Combination therapy with oral antihyperglycemic agents in the treatment fon non-insulin-dependent diabetes mellitus' TEM vol. 7, no. 6, 1996, pages 216 - 218
- ZSUZSA K. ET AL.: 'Rosiglitazone plus 7.5 mg glibenclamide improves glycemia in individuals with type 2 diabetes compared to increasing the glibenclamide dose to 15 mg' DIABETES vol. 52, no. 1, 2003, page A449

## Description

This invention relates to a pharmaceutical composition and in particular to a composition used in the treatment of diabetes mellitus, especially non-insulin dependent diabetes (NIDDM) (or Type 2 diabetes) and conditions associated with diabetes mellitus.

Insulin secretagogues are compounds that promote increased secretion of insulin by the pancreatic beta cells.

The sulphonylureas are well known examples of insulin secretagogues. The sulphonylureas act as hypoglycaemic agents and are used in the treatment of Type 2 diabetes. Examples of sulphonylureas include glibenclamide, glipizide, gliclazide, glimepiride, tolazamide and tolbutamide.

European Patent Application, Publication Number 0,306,228 relates to certain thiazolidinedione derivatives disclosed as having hypoglycaemic and hypolipidaemic activity. One particular thiazolidinedione disclosed in EP 0306228 is 5-[4-[2-(N-methyl-N-(2-pyridyl)amino)ethoxy]benzyl]thiazolidine-2,4-dione (hereinafter 'Compound (I)'). WO94/05659 discloses certain salts of Compound (I) including the maleate salt

Compound (I) is an example of a class of anti-hyperglycaemic agents known as 'insulin sensitisers'. In particular Compound (I) is a thiazolidanedione insulin sensitiser.

European Patent Applications, Publication Numbers: 0008203, 0139421, 0032128, 0428312, 0489663, 0155845, 0257781, 0208420, 0177353, 0319189, 0332331, 0332332, 0528734, 0508740; International Patent Application, Publication Numbers 92/18501, 93/02079, 93/22445 and United States Patent Numbers 5104888 and 5478852, also disclose certain thiazolidinedione insulin sensitisers.

Another series of compounds generally recognised as having insulin sensitiser activity are those typified by the compounds disclosed in International Patent Applications, Publication Numbers W093/2116G and WO94/01420. These compounds are herein referred to as'acyclic insulin sensitisers'. Other examples of acyclic insulin sensitisers are those disclosed in United States Patent Number 5232945 and International Patent Applications, Publication Numbers WO92/03425 and WO91/19702.

Examples of other insulin sensitisers are those disclosed in European Patent Application, Publication Number 0533933, Japanese Patent Application Publication Number 05271204 and United States Patent Number 5264451.

It is now surprisingly indicated that Compound (I) in combination with a sub maximal amount of an insulin secretagogue provides a particularly beneficial effect on glycaemic control, such combination is therefore particularly useful for the treatment of diabetes mellitus and conditions associated with diabetes. Lowering the dose of the insulin secretagogue in the presence of a full dose of insulin sensitising agent also has the benefit of reducing the likelihood, frequency and/or severity of hypoglycaemic episodes.

Accordingly, the invention provides a pharmaceutical composition comprising 2 to 8mg of 5-[4-[2-(N-methyl-N-(2-pyridyl)amino)ethoxy]benzyl]thiazolidine-2,4-dione (Compound I) or a pharmaceutically acceptable derivative thereof; a sub-maximal amount of an insulin secretagogue selected from: glibenclamide, glipizide, gliclazide, glimepiride, tolazamide and tolbutamide or repaglinide and a pharmaceutically acceptable carrier.

Particularly, the composition comprises 4mg of Compound (I), especially when administered per day.

Particularly, the composition comprises 4 to 8mg, especially when administered per day.

Preferably, the composition comprises 2 mg of Compound (I), especially when administered per day.

Preferably, the composition comprises 4 mg of Compound (I), especially when administered per day.

Preferably, the composition comprises 8 mg of Compound (I), especially when administered per day.

It will be understood that Compound (I) and the insulin secretagogue are each administered in a pharmaceutically acceptable form, including pharmaceutically acceptable derivatives such as pharmaceutically acceptable salts, esters and solvates thereof, as appropriate. In certain instances herein the names used for the relevant insulin secretagogues may relate to a particular pharmaceutical form of the relevant active agent: It will be understood that all pharmaceutically acceptable forms of the active agent per se are encompassed by this invention.

Suitable pharmaceutically acceptable salted forms of, Compound (I), include those described in the above mentioned patents and patent applications such as in EP 0306228 and WO94/05659 for Compound (I). A preferred pharmaceutically acceptable salt for Compound (I) is a maleate.

Suitable pharmaceutically acceptable solvated forms of Compound (I), include those described in the above mentioned patents and patent applications, such as in EP 0306228 and WO94/05659 for Compound (I), in particular hydrates.

Suitable pharmaceutically acceptable forms of the insulin secretagogue depend upon the particular compound used but include known pharmaceutically acceptable form of the particular compound chosen. Such derivatives are found or are referred to in standard reference texts such as the British and US Pharmacopoeias, Remington's Pharmaceutical Sciences (Mack Publishing Co.), Martindale The Extra Pharmacopoeia (London, The Pharmaceutical Press) (for example see the 31^{st} Edition page 341 and pages cited therein.)

Compound (I) and/or the pharmaceutically acceptable forms thereof, may be prepared using known methods, for example those disclosed in the above mentioned patents and patent applications, such as EP 0306228 and WO94/05659 for Compound (I). The disclosures of the above mentioned patents and patent applications, such as EP 0306228 and WO94/05659, are incorporated herein by reference.

Compound (I) may exist in one of several tautomeric forms, all of which are encompassed by the term Compound (I) as individual tautomeric forms or as mixtures thereof. Compound (I) contains a chiral carbon atom, and hence can exist in up to two stereoisomeric forms, the term Compound (I) encompasses all of these isomeric forms whether as individual isomers or as mixtures of isomers, including racemates.

The insulin secretagogue of choice is prepared according to known methods, such methods are found or are referred to in standard reference texts, such as the British and US Pharmacopoeias, Remington's Pharmaceutical Sciences (Mack Publishing Co.), Martindale The Extra Pharmacopoeia (London, The Pharmaceutical Press) (for example see the 31^{st} Edition page 341 and pages cited therein.)

When used herein the term'sub-maximal amount' of an insulin secretagogue means an amount lower than (that is less than 100% and typically within the range of from 5-95% of , for example 75%, 80%, 90% or 95% of) the appropriate non-combination dose for the insulin secretagogue in question, as described or referred to in reference texts such as the British National Formulary (BNF), British and US Pharmacopoeias, Remington's Pharmaceutical Sciences (Mack Publishing Co.), Martindale The Extra Pharmacopoeia (London, The Pharmaceutical Press). For example, for glibenclamide, the maximum dose quoted in the BNF is 15 mg daily; thus a sub-maximal amount of glibenclamide given together with an insulin sensitiser is typically 1.5-12.5 mg daily. For a second example, for gliclazide, the maximum daily dose quoted in the BNF is 320 mg daily; thus a sub-maximal amount of gliclazide given together with an insulin sensitiser is 20-300 mg daily. For a third example, for glipizide, the maximum dose quoted in the BNF is typically 40 mg daily; thus a sub-maximal amount of glipizide given together with an insulin sensitiser is typically 5-30 mg daily. For a fourth example, for tolazamide, the maximum dose quoted in the BNF is 1g daily; thus a sub-maximal amount of tolazamide given together with an insulin sensitiser is typically 50-950 mg daily. For a fifth example, for tolbutamide, the maximum dose quoted in the BNF is 2g daily, thus a sub-maximal amount of tolbutamide given together with an insulin sensitiser is typically 100 mg to 1.75g daily.

When used herein the term'conditions associated with diabetes' includes those conditions associated with diabetes mellitus itself and complications associated with diabetes mellitus.

'Conditions associated with diabetes mellitus itself include hyperglycaemia, insulin resistance, including acquired insulin resistance. Further conditions associated with diabetes mellitus itself include hypertension and cardiovascular disease, especially atherosclerosis and conditions associated with insulin resistance. Conditions associated with insulin resistance include polycystic ovarian syndrome and steroid induced insulin resistance and gestational diabetes.

'Complications associated with diabetes mellitus' includes renal disease, especially renal disease associated with Type 2 diabetes, neuropathy and retinopathy

Renal diseases associated with Type 2 diabetes include nephropathy, glomerulonephritis, glomerular sclerosis, hypertensive nephrosclerosis and end stage renal disease. Additional renal diseases associated with Type 2 diabetes include nephrotic syndrome.

As used herein the term pharmaceutically acceptable' embraces both human and veterinary use: for example the term 'pharmaceutically acceptable' embraces a veterinarily acceptable compound.

For the avoidance of doubt, when reference is made herein to scalar amounts, including mg amounts, of Compound (I) in a pharmaceutically acceptable form, the scalar amount referred to is made in respect of Compound (I) *per se:* For example 2 mg of Compound (I) in the form of the maleate salt is that amount of maleate salt which contains 2 mg of Compound (I).

Diabetes mellitus is preferably Type 2 diabetes.

Suitably the insulin sensitiser is the first administered agent.

In the present treatment the insulin sensitiser is administered at its normal, appropriate dose, for example Compound (I) is administered at a dose selected from 2-12mg per day, for example 1, 2, 4 or 8 mg per day.

Glycaemic control as referred to herein may be characterised using conventional methods, for example by measurement of a typically used index of glycaemic control such as fasting plasma glucose or glycosylated haemoglobin (HbAlc). Such indices are determined using standard methodology, for example those described in: Tuescher A, Richterich and P., Schweiz. med, Wschr. 101 (1971), 345 and 390 and Frank P., 'Monitoring the Diabetic Patent with Glycosolated Hemoglobin Measurements', Clinical Products 1988.

Compositions may be prepared by admixing an insulin sensitiser, such as Compound (I) and especially 2 to 8 mg thereof, a sub-maximal amount of an insulin secretagogue and a pharmaceutically acceptable carrier therefor.

Usually the compositions are adapted for oral administration. However, they may be adapted for other modes of administration, for example parenteral administration, sublingual or transdermal administration.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The compositions are preferably in a unit dosage form in an amount appropriate for the relevant daily dosage.

Suitable dosages for the insulin sensitisers include those disclosed in the abovementioned patents and patent applications.

Suitable dosages, including unit dosages, of Compound (I) comprise 1, 2, 3, 4, 5, 6, 7, 8, 9, 10, 11 or 12 mg of Compound (I).

Particular dosages of Compound (I) are 2mg/day, 4mg/day, including 2mg twice per day, and 8 mg/day, including 4mg twice per day.

In the treatment the medicaments may be administered from 1 to 6 times a day, but most preferably 1 or 2 times per day.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agent can be dissolved in the vehicle. To enhance the stability, the composition can be frozen after filling into the vial and the water removed under vacuum. Parenteral suspensions are prepared in substantially the same manner, except that the Compound (I) is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Compositions may contain from 0.1% to 99% by weight, preferably from 10-60% by weight, of the active material, depending upon the method of administration.

Composition may, if desired, be in the form of a pack accompanied by written or printed instructions for use.

The compositions are prepared and formulated according to conventional methods, such as those disclosed in standard reference texts, for example the British and US Pharmacopoeias, Remington's Pharmaceutical Sciences (Mack Publishing Co.), Martindale The Extra Pharmacopoeia (London, The Pharmaceutical Press) and Harry's Cosmeticology (Leonard Hill Books) (for example see the 31^{st} Edition page 341 and pages cited therein.)

The present invention also provides a pharmaceutical composition comprising 2 to 8mg of 5-[4-[2-(N-methyl-N-(2-pyridyl)amino)ethoxy]benzyl]thiazolidine-2,4-dione (Compound I) or a pharmaceutically acceptable derivative thereof; a sub-maximal amount of an insulin secretagogue selected from: glibenclamide, glipizide, gliclazide, glimepiride, tolazamide and tolbutamide or repaglinide.and a pharmaceutically acceptable carrier therefor, for use as an active therapeutic substance.

The invention also provides the use of a pharmaceutical composition comprising 2 to 8mg of 5-[4-[2-(N-methyl-N-(2-pyridyl)amino)ethoxy]benzyl]thiazolidine-2,4-dione (Compound I) or a pharmaceutically acceptable derivative thereof; a sub-maximal amount of an insulin secretagogue selected from: glibenclamide, glipizide, gliclazide, glimepiride, tolazamide and tolbutamide or repaglinide and a pharmaceutically acceptable carrier for the manufacture of a medicament for the treatment of diabetes mellitus and conditions associated with diabetes.

In particular, the present invention provides a pharmaceutical composition comprising comprising 2 to 8mg of 5-[4-[2-(N-methyl-N-(2-pyridyl)amino)ethoxy]benzyl]thiazolidine-2,4-dione (Compound I) or a pharmaceutically acceptable derivative thereof; a sub-maximal amount of an insulin secretagogue selected from: glibenclamide, glipizide, gliclazide, glimepiride, tolazamide and tolbutamide or repaglinide and a pharmaceutically acceptable carrier therefor, for use in the treatment of diabetes and conditions associated with diabetes.

A range of 2 to 4mg includes a range of 2.1 to 4, 2.2 to 4, 2.3 to 4, 2.4 to 4, 2.5 to 4, 2.6 to 4, 2.7 to 4.2.8 to 4, 2.9 to 4 or 3 to 4mg.

A range of 4 to 8mg includes a range of 4.1 to 8, 4.2 to 8, 4.3 to 8, 4.4 to 8, 4.5 to 8, 4.6 to 8,4.7 to 8, 4.8 to 8, 4.9 to 8, 5 to 8, 6 to 8 or 7 to 8mg.

A range of 8 to 12 mg includes a range of 8.1 to 12, 8.2 to 12, 8.3 to 12, 8.4 to 12, 8.5 to 12, 8.6 to 12, 8.7 to 12, 8.8 to 12, 8.9 to 12, 9 to 12, 10 to 12 or 11 to 12mg.

No adverse toxicological effects are expected for the compositions or methods of the invention in the above mentioned dosage ranges.

### Compositions of Compound (I)

**Preparation of Concentrate:** Tabletting concentrate was prepared using the following materials

| **Ingredient** | **Quantity (%)** |
|---|---|
| Milled Compound (I) as maleate salt | 13.25 (pure maleate salt) |
| | |
| Sodium Starch Glycollate | 5.00 |
| | |
| Hydoxypropyl Methylcellulose 2910 | 5.00 |
| | |
| Microcrystalline Cellulose | 20.0 |
| (Avicel PH102) | |
| | |
| Lactose Monohydrate, regular grade | to 100 |
| | |
| Purified water | * |

| | |
|---|---|
| * Removed during processing. | |

**The concentrate was then formulated into tablets using the following:**

| | **Quantity (mg per Tablet)** | | | |
|---|---|---|---|---|
| **Tablet Strength** | **1.0mg** | **2.0mg** | **4.0mg** | **8.0mg** |
| **Active Ingredient:** | | | | |
| Compound (I) maleate Concentrate granules | 10.00 | 20.00 | 40.00 | 80.00 |

| **Other Ingredients:** | | | | |
|---|---|---|---|---|
| Sodium Starch Glycollate | 6.96 | 6.46 | 5.46 | 10.92 |
| Microcrystalline Cellulose (Avicel PH102) | 27.85 | 25.85 | 21.85 | 43.70 |
| Lactose monohydrate, (Pharmatose DCL15), | 104.44 | 96.94 | 81.94 | 163.88 |
| Magnesium Stearate | 0.75 | 0.75 | 0.75 | 1.50 |
| **Total Weight of Tablet Core** | 150.0 | 150.0 | 150.0 | 300.0 |
| Opadry | 4.5 | 4.5 | 4.5 | 9.0 |
| | | | | |
| **Total Weight of Film Coated Tablet** | 154.5 | 154.5 | 154.5 | 309.0 |

Compositions for other active agents are as described in the above mentioned publications.

## Claims

1. A pharmaceutical composition comprising 2 to 8mg of 5-[4-[2-(N-methyl-N-(2-pyridyl)amino)ethoxy]benzyl]tiazolidine-2,4-dione(Compound I) or **a pharmaceutically acceptable derivative thereof;** a sub maximal amount of an insulin secretagogue selected from: glibenclamide, glipizide, gliclazide, glimepiride, tolazamide and tolbutamide or repaglinide and a pharmaceutically acceptable carrier.

2. A composition according to claim 1, which comprises 2 to 4 or 4 to 8 mg of 5-[4-[2-(N-methyl-N-(2-pyridyl)amino)ethoxy]benzyl]thiazolidine-2,4-dione (Compound I) or **a pharmaceutically acceptable derivative thereof.**

3. A composition according to claim 1, which comprises 2 to 4mg of 5-[4-[2-(N-methyl N-(2 pyridyl)amino)ethoxy]benayl]thiazolidine-2,4-dione (Compound I) or **a pharmaceutically acceptable derivative thereof.**

4. A composition according to claim 1, which comprises 4 to 8mg of 5-[4-[2-(N-methyl-N-(2-pyridyl)amino)ethoxy]benzyl]thiazolidine-2,4-dione (Compound I) or **a pharmaceutically acceptable derivative thereof.**

5. A composition according to claim 1, which comprises 2 mg of 5-[4-[2-(N-methyl-N-(2-pyzidyl)amino)ethoxy]benzyl]thiazolidine-2,4-dione (Compound 1) or **a pharmaceutically acceptable derivative thereof.**

6. A composition according to claim 1, which comprises 4 mg of 5-[4-[2-(N-methyl-N-(2-pyridyl)amino)ethoxy]benzyl]thiazolidine-2,4-dione (Compound I) or **a pharmaceutically acceptable derivative thereof.**

7. A composition according to claim 1, which comprises 8 mg of 5-[4-[2-(N-methyl-N-(2-pyridyl)amino)ethoxy]benzyl]thiazolidino-2,4-dione (Compound I) or **a pharmaceutically acceptable derivative thereof.**

8. A pharmaceutical composition comprising 2 to 8mg of 5-[4-[2-(N-methyl-N-(2-pyridyl)amino)ethoxy]benzyl]thiazolidine-2,4-dione (Compound I) or **a pharmaceutically acceptable derivative thereof;** a sub-maximal amount of an insulin secretagogue selected from: glibenclamide, glipizide, gliclazide, glimepiride, tolazamide and tolbutamide or repaglinide and a pharmaceutically acceptable carrier, for use as an active therapeutic substance

9. A pharmaceutical composition comprising 2 to 8mg of 5-[4-[2-(N-methyl-N-(2-pyridyl)amino)ethoxy]benzyl]thiazolidine-2,4-dione (Compound I) or **a pharmaceutically acceptable derivative thereof;** a sub-maximal amount of an insulin secretagogue selected from: glibenclamide, glipizide, gliclazide, glimepiride, tolazamide and tolbutamide or repaglinide and a pharmaceutically acceptable carrier, for use in the treatment of diabetes mellitus and conditions associated with diabetes mellitus.

10. A pharmaceutical composition comprising 2 to 8mg of 5-[4-[2-(N-methyl-N-(2-pyridyl)amino)ethoxy]benzyl]thiazolidine-2,4-dione (Compound 1) or **a pharmaceutically acceptable derivative thereof;** a sub-maximal amount of an insulin secretagogue selected from: glibenclamide, glipizide, gliclazide, glimepiride, tolazamide and tolbutamide or repaglinide and a pharmaceutically acceptable carrier in unit dose form.

11. Use of a pharmaceutical composition comprising 2 to 8mg of 5-[4-[2-(N-methyl-N-(2-pyridyl)amino)ethoxy]benzyl]thiazolidine-2,4-dione (Compound I) or **a pharmaceutically acceptable derivative thereof;** a sub-maximal amount of an insulin secretagogue selected from: glibenclamide, glipizide, gliclazide, glimepiride, tolazamide and tolbutamide or repaglinide and a pharmaceutically acceptable carrier for the manufacture of a medicament for the treatment of diabetes mellitus and conditions associated with diabetes mellitus.

12. A pharmaceutical composition according to any one of claims 1 to 10 or use according to claim 11 wherein Compound I is present as a salt.

13. A pharmaceutical composition according to any one of claims 1 to 10 or use according to claim 11 wherein Compound I is present as a maleate salt.

## Patentansprüche

1. Arzneimittel, umfassend 2 bis 8 mg 5-[4-[2-(N-Methyl-N-(2-pyridyl)amino)ethoxy]benzyl]thiazolidin-2,4-dion (Verbindung I) oder ein pharmazeutisch verträgliches Derivat davon, eine submaximale Menge eines Insulin-Sekretagogens, ausgewählt aus Glibenclamid, Glipizid, Gliclazid, Glimepirid, Tolazamid und Tolbutamid oder Repaglinid, und einen pharmazeutisch verträglichen Träger.

2. Zusammensetzung nach Anspruch 1, welche 2 bis 4 oder 4 bis 8 mg 5-[4-[2-(N-Methyl-N-(2-pyridyl)amino)ethoxy]benzyl]thiazolidin-2,4-dion (Verbindung I) oder ein pharmazeutisch verträgliches Derivat davon umfasst.

3. Zusammensetzung nach Anspruch 1, welche 2 bis 4 mg 5-[4-[2-(N-Methyl-N-(2-pyridyl)amino)ethoxy]benzyl]thiazolidin-2,4-dion (Verbindung I) oder ein pharmazeutisch verträgliches Derivat davon umfasst.

4. Zusammensetzung nach Anspruch 1, welche 4 bis 8 mg 5-[4-[2-(N-Methyl-N-(2-pyridyl)amino)ethoxy]benzyl]thiazolidin-2,4-dion (Verbindung 1) oder eines pharmazeutisch verträglichen Derivats davon umfasst.

5. Zusammensetzung nach Anspruch 1, welche 2 mg 5-[4-[2-(N-Methyl-N-(2-pyridyl)amino)ethoxy]benzyl]thiazolidin-2,4-dion (Verbindung I) oder eines pharmazeutisch verträglichen Derivats davon umfasst.

6. Zusammensetzung nach Anspruch 1, welche 4 mg 5-[4-[2-(N-Methyl-N-(2-pyridyl)amino)ethoxy]benzyl]thiazolidin-2,4-dion (Verbindung I) oder eines pharmazeutisch verträglichen Derivats davon umfasst.

7. Zusammensetzung nach Anspruch 1, welche 8 mg 5-[4-[2-(N-Methyl-N-(2-pyridyl)amino)ethoxy]benzyl]thiazolidin-2,4-dion (Verbindung I) oder eines pharmazeutisch verträglichen Derivats davon umfasst.

8. Arzneimittel, umfassend 2 bis 8 mg 5-[4-[2-(N-Methyl-N-(2-pyridyl)amino)ethoxy]benzyl]thiazolidin-2,4-dion (Verbindung I) oder ein pharmazeutisch verträgliches Derivat davon, eine submaximale Menge eines Insulin-Sekretagogens, ausgewählt aus Glibenclamid, Glipizid, Gliclazid, Glimepirid, Tolazamid und Tolbutamid oder Repaglinid, und einen pharmazeutisch verträglichen Träger zur Verwendung als therapeutischer Wirkstoff.

9. Arzneimittel, umfassend 2 bis 8 mg 5-[4-[2-(N-Methyl-N-(2-pyridyl)amino)ethoxy]benzyl]thiazolidin-2,4-dion (Verbindung I) oder ein pharmazeutisch verträgliches Derivat davon, eine submaximale Menge eines Insulin-Sekretagogens, ausgewählt aus Glibenclamid, Glipizid, Gliclazid, Glimepirid, Tolazamid und Tolbutamid oder Repaglinid, und einen pharmazeutisch verträglichen Träger zur Verwendung bei der Behandlung von Diabetes mellitus und mit Diabetes mellitus verbundenen Zuständen.

10. Arzneimittel, umfassend 2 bis 8 mg 5-[4-[2-(N-Methyl-N-(2-pyridyl)amino)ethoxy]benzyl]thiazolidin-2,4-dion (Verbindung I) oder ein pharmazeutisch verträgliches Derivat davon, eine submaximale Menge eines Insulin-Sekretagogens, ausgewählt aus Glibenclamid, Glipizid, Gliclazid, Glimepirid, Tolazamid und Tolbutamid oder Repaglinid, und einen pharmazeutisch verträglichen Träger in Einheitsdosierungsform.

11. Verwendung eines Arzneimittels, umfassend 2 bis 8 mg 5-[4-[2-(N-Methyl-N-(2-pyridyl)amino)ethoxy]benzyl]thiazolidin-2,4-dion (Verbindung I) oder ein pharmazeutisch verträgliches Derivat davon, eine submaximale Menge eines Insulin-Sekretagogens, ausgewählt aus Glibenclamid, Glipizid, Gliclazid, Glimepirid, Tolazamid und Tolbutamid oder Repaglinid, und einen pharmazeutisch verträglichen Träger zur Herstellung eines Medikaments zur Behandlung von Diabetes mellitus und mit Diabetes mellitus verbundenen Zuständen.

12. Arzneimittel nach einem der Ansprüche 1 bis 10 oder Verwendung nach Anspruch 11, wobei Verbindung I als Salz vorliegt.

13. Arzneimittel nach einem der Ansprüche 1 bis 10 oder Verwendung nach Anspruch 11, wobei Verbindung I als Maleatsalz vorliegt.

## Revendications

1. Composition pharmaceutique comprenant 2 à 8 mg de 5-[4-[2-(N-méthyl-N-(2-pyridyl)amino)éthoxy]benzyl]thiazolidine-2,4-dione (Composé I) ou d'un de ses dérivés pharmaceutiquement acceptables ; une quantité sous-maximale d'un sécrétagogue d'insuline choisi entre : le glibenclamide, le glipizide, le gliclazide, le glimépiride, le tolazamide et le tolbutamide ou le répaglinide et un support pharmaceutiquement acceptable.

2. Composition suivant la revendication 1, qui comprend 2 à 4 ou 4 à 8 mg de 5-[4-[2-(N-méthyl-N-(2-pyridyl)amino)éthoxy]benzyl]thiazolidine-2,4-dione (Composé I) ou d'un de ses dérivés pharmaceutiquement acceptables.

3. Composition suivant la revendication 1, qui comprend 2 à 4 mg de 5-[4-[2-(N-méthyl-N-(2-pyridyl)amino)-éthoxy]benzyl]thiazolidine-2,4-dione (Composé I) ou d'un de ses dérivés pharmaceutiquement acceptables.

4. Composition suivant la revendication 1, qui comprend 4 à 8 mg de 5-[4-[2-(N-méthyl-N-(2-pyridyl)amino)éthoxy]benzyl]thiazolidine-2,4-dione (Composé I) ou d'un de ses dérivés pharmaceutiquement acceptables.

5. Composition suivant la revendication 1, qui comprend 2 mg de 5-[4-[2-(N-méthyl-N-(2-pyridyl)amino)éthoxy]benzyl]thiazolidine-2,4-dione (Composé I) ou d'un de ses dérivés pharmaceutiquement acceptables.

6. Composition suivant la revendication 1, qui comprend 4 mg de 5-[4-[2-(N-méthyl-N-(2-pyridyl)amino)éthoxy]benzyl]thiazolidine-2,4-dione (Composé I) ou d'un de ses dérivés pharmaceutiquement acceptables.

7. Composition suivant la revendication 1, qui comprend 8 mg de 5-[4-[2-(N-méthyl-N-(2-pyridyl)amino)éthoxy]benzyl]thiazolidine-2,4-dione (Composé I) ou d'un de ses dérivés pharmaceutiquement acceptables.

8. Composition pharmaceutique comprenant 2 à 8 mg de 5-[4-[2-(N-méthyl-N-(2-pyridyl)amino)éthoxy]benzyl]thiazolidine-2,4-dione (Composé I) ou d'un de ses dérivés pharmaceutiquement acceptables ; une quantité sous-maximale d'un sécrétagogue d'insuline choisi entre : le glibenclamide, le glipizide, le gliclazide, le glimépiride, le tolazamide et le tolbutamide ou le répaglinide et un support pharmaceutiquement acceptable, destiné à être utilisé comme substance thérapeutique active.

9. Composition pharmaceutique comprenant 2 à 8 mg de 5-[4-[2-(N-méthyl-N-(2-pyridyl)amino)éthoxy]benzyl]thiazolidine-2,4-dione (Composé I) ou d'un de ses dérivés pharmaceutiquement acceptables ; une quantité sous-maximale d'un sécrétagogue d'insuline choisi entre : le glibenclamide, le glipizide, le gliclazide, le glimépiride, le tolazamide et le tolbutamide ou le répaglinide et un support pharmaceutiquement acceptable, destiné à être utilisé dans le traitement du diabète sucré et d'affections associées au diabète sucré.

10. Composition pharmaceutique comprenant 2 à 8 mg de 5-[4-[2-(N-méthyl-N-(2-pyridyl)amino)éthoxy]benzyl]thiazolidine-2,4-dione (Composé I) ou d'un de ses dérivés pharmaceutiquement acceptables ; une quantité sous-maximale d'un sécrétagogue d'insuline choisi entre : le glibenclamide, le glipizide, le gliclazide, le glimépiride, le tolazamide et le tolbutamide ou le répaglinide et un support pharmaceutiquement acceptable, dans une forme posologique unitaire.

11. Utilisation d'une composition pharmaceutique comprenant 2 à 8 mg de 5-[4-[2-(N-méthyl-N-(2-pyridyl)-amino)éthoxy]benzyl]thiazolidine-2,4-dione (Composé I) ou d'un de ses dérivés pharmaceutiquement acceptables ; une quantité sous-maximale d'un sécrétagogue d'insuline choisi entre : le glibenclamide, le glipizide, le gliclazide, le glimépiride, le tolazamide et le tolbutamide ou le répaglinide et un support pharmaceutiquement acceptable, pour la production d'un médicament destiné au traitement du diabète sucré et d'affections associées au diabète sucré.

12. Composition pharmaceutique suivant l'une quelconque des revendications 1 à 10 ou utilisation suivant la revendication 11, dans laquelle le Composé I est présent sous forme d'un sel.

13. Composition pharmaceutique suivant l'une quelconque des revendications 1 à 10 ou utilisation suivant la revendication 11, dans laquelle le Composé I est présent sous forme d'un maléate.
